# EUROPEAN PATENT SPECIFICATION

(11) **EP 1 853 545 B1**
(45) Date of publication and mention of the grant of the patent: **26.05.2010**
(21) Application number: 06708307.1
(22) Date of filing: 16.02.2006
(51) Int. Cl.: C07C 61/37, C07C 51/487, C07C 323/32

(54) **PROCESS FOR OBTAINING ENANTIOMERS OF CHRYSANTHEMIC ACID**
VERFAHREN ZUR GEWINNUNG VON ENANTIOMEREN VON CHRYSANTHEMSÄURE
PROCEDE DE PRODUCTION D'ENANTIOMERES D'ACIDE CHRYSANTHEMIQUE

(30) Priority: 17.02.2005 IT MI20050231
(43) Date of publication of application: 14.11.2007
(73) Proprietor: ENDURA S.p.A., I-40121 Bologna (IT)
(72) Inventor: BORZATTA, Valerio, I-40127 Bologna (IT); ROSINI, Goffredo, I-40135 Bologna (IT); AYOUB, Claudia, I-10067 Rastignano (IT); RIGHI, Paolo, 40139 BOLOGNA (IT); CAPPARELLA, Elisa, I-48100 Ravenna (IT)
(74) Representative: Gervasi, Gemma
(86) International application number: PCT/EP2006/060013
(87) International publication number: WO 2006/087357

(56) References cited:
- US-A- 4 257 976
- DATABASE BEILSTEIN BEILSTEIN INSTITUTE FOR ORGANIC CHEMISTRY, FRANKFURT-MAIN, DE; XP002388384 Database accession no. 2098529 & KINISHI ET AL: AGRIC. BIOL. CHEM., vol. 42, 1978, page 869,
- ISHBEL G.M. ET AL: "The Chrysanthemuncarboxylic acids. IV.-Optical Resolution of the Chrysanthemic Acids" J. SCI. FOOD A, vol. 3, 1952, pages 189-192, XP002388381 cited in the application
- ÉVA KOZSDA-KOVÁCS: "role of the solvent in optical resolution of trans-chrysanthemic acid via diastereomeric salt formation with (1R,2R)-1-(4-nitro-phenyl)-2-dimethylamino propane-1,3-diol" J. CHEM. SOC., PERKIN TRANS, vol. 2, 2000, pages 149-153, XP002388382 cited in the application

## Description

### FIELD OF THE INVENTION

The present invention relates to the field of pyrethroid insecticides and preparation and purification procedures thereof. A new process is described for obtaining enantiomers of chrysanthemic acid.

### PRIOR ART

Within the last thirty years much work has been carried out in the field of 2,2-dimethyl-3-(2-methyl-1-propenyl)cyclopropanecarboxylic acid synthesis (Chrysanthemic acid, here abbreviated as ChA). This compound is an important starting material for the synthesis of various pyrethroid insecticides, widely used in agriculture and domestically for controlling ants, spiders, mosquitoes, flies and other undesirable insects; they are characterised by having a low toxicity towards mammals and a high insecticidal activity.

Chrysanthemic acid is a chiral compound, containing centres of asymmetry and is optically active: it can be present in the four forms d-*cis*, I-*cis*, *d-trans*, I-*trans*, characterised by varying optical and geometric isomerism, and in the respective mixtures.

Commercially available racemic ChA is obtained mainly by non-specific synthesis (Schechter, M. S. et al J. Am. Chem. Soc. 1949, 71, 3165; US 2,574,500/1951; Martel, J., Huynh, C. Bull. Soc. Chim. France 1967, 985) and is present typically as a *trans*/*cis* mixture, particularly in the ratios 65/35, 80/20, 92/8. The *trans* isomer, in particular the *d-trans* configuration, is preferred as it possesses greater insecticidal activity; further work has therefore been undertaken to obtain chrysanthemic acid particularly in the d-*trans* configuration.

ChA mixtures enriched in the d- enantiomer are currently obtainable by applying known methods of asymmetric synthesis (Nozaki, H. et al. Tetrahedron Lett. 1966, 7, 5239; Aratani, T. et al. Tetrahedron Lett. 1975, 16, 1707; *ibid*. 1977, 18, 2599; *ibid*. 1982, 23, 685; Aratani, T. Pure Appl. Chem. 1985, 57, 1839; Lowental, R. E. Masamune, S. Tetrahedron Lett. 1991, 32, 7373; Masamune, S., Lowental, R. E.-US 5 298 623, 1994; Kanemasa, S. et al. Tetrahedron Lett. 1994, 35, 7985; Evans, D. A. et al. J. Am. Chem. Soc. 1991, 113, 726; Evans, D. A. et al Angew. Chem. Int. Ed. Engl. 1992, 31, 430; Sanders, C. J. et al. Tetrahedron: Asymmetry 2001, 12, 1055); however many of these methods are rather complex, costly and hard to develop on an industrial scale: particularly indicated are problems of expense, instability and recovery of the diastereo- and enantio- selective catalyst, with the need to use complex and dedicated equipment and to operate at very low temperatures with reagents and solvents of high purity. For these reasons, on an industrial level, the production of ChA by non-specific synthesis (racemic or scalemic) is still preferred, with recovery of the required isomer from the obtained isomeric mixtures by selective precipitation processes (resolution) of the diastereoisomeric salts. To separate the *cis*/*trans* diastereoisomers of chrysanthemic acid, the following are commonly used: (a) repeated fractional crystallizations, (b) conversion of the *cis* isomer into lactones or other derivatives in an attempt to better distinguish its physico-chemical and/or solubility characteristics from those of the *trans* isomer, or even (c) enrichment of the more stabile *trans* isomer by means of isomerization reactions. The yield of these processes is known to vary significantly depending on the *cis*/*trans* ratio of the starting mixture and the number of steps necessary for the differentiation and isomerization processes.

For resolution of chrysanthemic acid d- and I- enantiomers, some chiral bases are utilized which form salts with the two enantiomers of varying solubility in specific solvents or solvent mixtures; of the chiral bases utilized, the following can be mentioned: quinine (J.Sci.Food.Agric. 1952, 3, 189-192), lysine (Agr.Biol.Chem., 1971, 35, 1984), L-2-benzyl-aminopropanol (Agr.Biol.Chem., 1973, 37, 1713), D-N-methyl-ephedrine (US 4,257,976), and 2-dimethylamino-1-(4-nitrophenyl)propanediol (DMAD) in mixtures of ether and methanol (FR 1481978).

With regard to DMAD, recent studies (J.Chem.Soc.Perkin., Trans.2, 2000, 149) show that methanol is indispensable for promoting nucleation and growth in crystals of the less soluble diastereoisomeric salt; the same studies show that methanol molecules remain in the final crystal. Incorporation of the solvent increases toxicity to man and reduces activity per unit weight of the crystalline compound thus obtained.

The need to use a mixture of solvents of precise composition is an obstacle to the effective recovery of the solvents utilized and their use in subsequent processes, with strong repercussions on costs and on production times.

In the light of the known art, the need exists for improved processes for separation and recovery of the desired *trans*-chrysanthemic acid enantiomers, particularly the d-*trans* enantiomer, from mixtures of its diastereoisomers and enantiomers, obtained by common non-specific or asymmetric synthesis processes; also desirable would be to obtain this product at high yields without recourse to using solvent mixtures difficult to recover and reuse, or toxic solvents. Finally, it would be desirable to obtain a method for separating chrysanthemic acid enantiomers with an effectiveness independent of the diastereoisomeric and enantiomeric composition of the starting mixture, and particularly independent of the quantity of *cis*-ChA present in the mixture.

### SUMMARY

The present invention concerns a new process for separating and recovering d-trans-chrysanthemic acid (d-*trans*-ChA) and/or I-*trans*-chrysanthemic *acid* (I-*trans-*ChA) from mixtures of isomers of said acid; said starting mixtures can contain the two d- and I- enantiomers in an equal quantity (racemic mixtures) or can be enantiomerically enriched in one of the two d- or I- forms (scalemic mixtures).

The process is characterised by the use of two chiral selectors: 2-dimethylamino-1-phenyl-1,3-propanediol (DMPP) and 2-dimethylamino-1-[4-(methylthio)phenyl} propane-1,3-diol (MTDP); these compounds are utilized in one of their enantiomeric forms 1 S,2S-(+) or 1R,2R-(-).

DMPP has the ability to form salts selectively with *trans*-ChA enantiomers, which are then separated by precipitation, while being inactive with *cis*-ChA which remains in solution.

MTDP has the ability to precipitate only one of the two enantiomers (d-*trans*-ChA or I-*trans*-ChA) in salt form, leaving the respective counter enantiomer in solution.

ChA free acids are easily displaced from their salts with DMPP and MTDP by treating with acid solutions. The bases DMPP and MTDP are recovered enantiomerically intact by alkalinization and can be reused in further process cycles.

The invention includes chrysanthemic acid salts with the aforesaid chiral selectors as intermediates of the process, and the MTDP selector as such, synthesised as a new molecule by the Applicants.

The process described herein can be carried out in a single solvent rather than in solvent mixture, which are difficult to recover and reuse, and the final crystallized product does not incorporate molecules of solvent.

### DETAILED DESCRIPTION OF THE INVENTION

In the present invention DMPP is utilized in one of its enantiomeric forms 1*S*,2*S-*(+) or *1R*,*2R*-(-) to separate *trans*-ChA from *cis*-ChA by selective precipitation of the *trans* diastereoisomer: indeed DMPP, utilized in a stoichiometric quantity (1:1) relative to the *trans*-ChA present in the mixture, reacts selectively with this latter to form the salt of *trans*-ChA with DMPP as precipitate; said salt will be respectively *trans*-ChA•*1S*,*2S*-(+)-DMPP, or *trans*-ChA•*1R,2R*-(-)-DMPP, depending on whether *1S*,*2S-*(+)-DMPP or *1R*,*2R*-(-)-DMPP is used as the chiral selector.

The two aforesaid salts belong to the salt category *p₁n₁* because they are the co-crystallization product of the two enantiomers d-*trans*-ChA and I-*trans*-ChA in a 1:1 ratio. DMPP, in the stoichiometric ratios used, does not react with the *cis*-ChA present in the initial mixture and remains in solution; therefore by separating the *p₁n₁* salt precipitated from the mother liquors, *trans*-ChA is separated from *cis-*ChA.

The *p₁n₁* s precipitated salt, separated from the mother liquors, is then treated with an acidic solution: in this manner the d-*trans*-ChA and I-*trans*-ChA enantiomers are displaced from the salt and returned to the solution. The acidic solution is then extracted with an organic solvent: the d-*trans* and I-*trans* enantiomers pass into the organic phase from where they are recovered in solid form by removing the solvent; the DMPP amine, having remained in the aqueous phase in the form of an ammonium salt, can be precipitated by basification: the DMPP amine is obtained in an enantiomerically intact form which can be recycled in later stages of the process.

The d-*trans*-ChA and I-*trans*-ChA mixture thus recovered is dissolved in a suitable solvent and then treated with MTDP. This amine is used for selectively precipitating only one of the two *trans*-ChA enantiomers in the form of a salt, leaving the counter enantiomer in solution. In particular, if *1S*,*2S*-(+)-MTDP is used in a stoichiometric quantity (1:1) relative to the I-*trans*-ChA enantiomer present in the mixture (i.e. 0.5 equivalents relative to total *trans*-ChA), the salt that forms and precipitates is I-*trans*-ChA•*1S*,*2S*-(+)-MTDP, whereas the counter enantiomer d-*trans*-ChA remains in solution. If *1R*,*2R*-(-)-MTDP is used instead, in the same stoichiometric quantity (1:1) relative to the d-*trans*-ChA enantiomer present in the mixture (i.e. 0.5 equivalents relative to total *trans*-ChA), the salt that forms and precipitates is d-*trans*-ChA•*1R*,*2R*-(-)-MTDP, whereas the counter enantiomer I-*trans*-ChA remains in solution.

The two aforementioned salts belong to the salt category *n* because they consist of a single enantiomer (d- or I-) salified with MTDP. By separating the *n* salt from the mother liquors, d-*trans*-ChA is separated from I-*trans*-ChA.

The enantiomer salified with MTDP can be displaced from its *n* salt by treating with an acid solution and extracting with an organic solvent in a similar manner to the aforedescribed method: the enantiomer is obtained in the solid state by evaporating the organic phase unitl to dryness; the MTDP amine remains in the aqueous phase from where it can be precipitated by basification: the MTDP amine is obtained in an enantiomerically intact form which can be recycled in later stages of the process.

The counter enantiomer remaining in solution is recovered in the solid state by filtering the solution from the *n* salt, then evaporating the filtered solution to dryness.

The residual mother liquors from the preceding reaction with DMPP, being rich in *cis*-ChA, can be treated in their turn as follows, to obtain an additional quantity of d-*trans* and/or I-*trans*-ChA: they can be subjected to epimerisation by treating with Lewis acids as described in EP62979 or by racemization as described in US 4,898,655 with the preferential conversion of *cis*-ChA to *trans*-ChA. The resulting *trans*-ChA solution is then reacted with MTDP as in the previously described methods until d-*trans*-ChA and/or I-*trans*-ChA are obtained in the solid form.

The aforesaid reactions with DMPP and MTDP have been described starting from racemic mixtures of ChA, i.e. containing equal quantities of d-ChA and I-ChA. However, this amine pair also enables d-*trans*-ChA and I-*trans*-ChA enantiomers to be obtained starting from scalemic mixtures of ChA, i.e. enriched in the d- or I-enantiomer.

In this case the process is undertaken in one of the four following manners; in the case of scalemic mixtures of ChA **enriched in d-:**
a) the mixture is treated with 1*S*,2*S*-(+)-DMPP in a quantity of 2 equivalents relative to the lesser enantiomer (I-) present in the mixture; the *p₁n₁* salt is obtained as precipitate; the salification mother liquors also contain, in addition to *cis*-ChA, the excess enantiomer (d-) which did not react with DMPP.
b) the mixture is treated with *1R*,*2R*-(-)-DMPP in a quantity of 1 equivalent relative to the excess enantiomer present (d-); in this case it is the enantiomeric excess which precipitates with DMPP in the n salt form; the salification mother liquors also contain, in addition to *cis*-ChA, the d-*trans*-*ChA* and I-*trans-ChA* racemic mixture which did not react with DMPP.

In the case of the scalemic mixture of ChA **enriched in I-:**
c) the mixture is treated with 1,R,2R-(-)-DMPP in a quantity of 2 equivalents relative to the lesser enantiomer (d-) present in the mixture; the *p₁n₁* salt is obtained as precipitate; the salification mother liquors also contain, in addition to *cis*-ChA, the excess enantiomer (I-) which did not react with DMPP.
d) the mixture is treated with *1S*,*2S*-(+)-DMPP in a quantity of 1 equivalent relative to the excess enantiomer (I-) present; in this case the enantiomeric excess precipitates with DMPP in the *n* salt form; the salification mother liquors also contain, in addition to *cis*-ChA, the d-*trans-ChA* and I-*trans*-*ChA* racemic mixture which did not react with DMPP.

In case a) the excess d- enantiomer remaining in solution is recovered by reacting this latter with *1R*,*2R*-(-)-DMPP in a stoichiometric quantity (1:1) relative to the aforesaid excess d- enantiomer; this precipitates quantitatively in the *n* salt form, while *cis*-ChA remains in solution.

In case c) the excess I- enantiomer remaining in solution is recovered by reacting this latter with *1S*,*2S*-(+)-DMPP in a stoichiometric quantity (1:1) relative to the aforesaid excess I- enantiomer; this precipitates quantitatively in the *n* salt form, while *cis*-ChA remains in solution.

In all cases a), b), c) and d) where a *p₁n₁* salt is obtained, the enantiomers contained therein are always recovered as previously described i.e. by treating the salt with acids, extracting with organic solvent and evaporating the organic phase to dryness; the solid obtained is redissolved in water and treated with MTDP until the d-*trans* and/or I-*trans* enantiomers are recovered in solid form. Where an *n* salt is obtained, the enantiomer contained therein is always recovered as previously described, i.e. by treating the salt with acids, extracting the acid solution with an organic solvent then evaporating the organic phase to dryness.

The present invention has enabled d-*trans*.-ChA and/or I-*trans*-ChA to be obtained starting from any ChA i.e. in any one of its racemic or scalemic mixtures.

From the diastereoisomeric composition viewpoint, all ChA forms in all possible *trans*/*cis* ratios are included, for example 65/35, 80/20 and 92/8, which are commonly available commercially. It has indeed been observed that the *trans*/*cis* ratio in the starting mixture does not influence the yield of the present process (considered as the quantity of recovered isomer relative to the quantity of said isomer present in the starting mixture); if d*-trans*-ChA is to be produced in high quantity, it would in any case be useful to start from ChA mixtures more enriched in the *trans* isomer.

From the enantiomeric composition viewpoint, the starting ChA can be enantiomerically non-enriched (as in the case of ChA produced in a non-stereospecific manner); alternatively it can be enriched in the d- or I- enantiomer (as obtainable by synthesis by asymmetric catalysis).

Both reaction passages with DMPP and MTDP are preferably undertaken in isopropyl ether; in both cases, using this solvent enables salification and hence selective precipitation of the desired isomer; precipitation takes place rapidly and consistently, in a manner that is broadly independent of reaction temperature, and without the need to add co-solvents such as nucleation agents. The process can comprise the use of any other solvent which has a dissolving/precipitating capacity towards the aforesaid ChA isomers substantially equivalent to those of isopropyl ether, for example toluene, t-butylmethylether, tetrahydrofuran, 1,2-dimethoxyethane.

All the aforesaid salification reactions with DMPP take place preferably at a temperature between 0°C and 100°C for a period of between 10 minutes and 5 hours; more preferably at a temperature between 50°C and 80°C for a period of between 1 minute and 1 hour, or even more preferably between 10 minutes and 30 minutes.

In a preferred embodiment of the method, the DMPP is previously mixed with isopropyl ether and the mixture heated at reflux; ChA which has been previously dissolved in isopropyl ether is added to this mixture and the resulting mixture is maintained at reflux under agitation.

The salt thus precipitated with DMPP is separated from the mixture and is washed in a suitable manner, preferably with isopropyl ether; the salt obtained is in *trans* form and is substantially pure (about 98%); small residual amounts of *cis* diastereiosomer can be removed by additional washes with isopropyl ether and/or crystallization from solvent (e.g. toluene).

The reaction with MTDP takes place preferably in isopropyl ether, at a temperature between 0°C and 70°C for a period of between 10 minutes and 5 hours, more preferably at a temperature between 50°C and 70°C for a period of between 10 minutes and 1 hour.

In a preferred embodiment of the method, MTDP is previously dissolved in isopropyl ether and heated under reflux; d,I-*trans*-ChA is then added, also predissolved in isopropyl ether, and the resulting mixture is maintained at reflux under agitation. The salt with MTDP, suitably washed one or more times with isopropyl ether provides, after acidification, d-*trans*-ChA with an enantiomeric excess equal to about 95%. This enantiomeric excess can be increased if required by additional washes and recrystallizations of the salt.

The type of acid used to displace ChA from its salts with DMPP or MTDP is not specified; the solvent used for extraction is generally an organic solvent such as isopropyl ether, toluene, chloroform, methylene chloride or ethyl acetate; isopropyl ether is preferred.

A further aspect of the invention is the provision of, as such:
the following *p₁ n₁* salts with DMPP:
   dl-*trans*-ChA•*1S*,*2S*-(+)-2-dimethylamino-1-phenyl-1,3-propanediol;
   dl-*trans*-ChA•*1R*,*2R*-(-)-2-dimethylamino-1-phenyl-1,3-propanediol;
the following *n* salts with DMPP:
   I-*trans*-ChA•*1S*,*2S*-(+)-2-dimethylamino-1-phenyl-1,3-propanediol;
   d-*trans*-ChA•1*R*,2*R*-(-)-2-dimethylamino-1-phenyl-1,3-propanediol, the following n salts with MTDP:
   I-trans-ChA•1*S*,2*S*-(+)-2-dimethylamino-1-[4-(methylthio)phenyl]propane-1,3-diol;
   d-*trans*-ChA•1*R*,2*R*-(-)-2-dimethylamino-1-[4-(methylthio)phenyl]propane-1,3-diol.

All the aforesaid salts are homogeneous microcrystalline solids characterised by specific physico-chemical and spectroscopic values (mp, [α]_{D}, IR, ¹H NMR, ¹³C, NMR, Mass) as highlighted in the experimental part. These values are characteristics of the aforesaid salts, as indicated by their remaining unchanged after redissolving and recrystallization of the product.

Studies by the Applicant have shown that the aforesaid *p₁ n₁* salts are the product of co-crystallization in a fixed 1:1 proportion of d-*trans*-ChA•*1S*,*2S*-(+)-(DMPP) and I-*trans*•*1S*,*2S*-(+)-(DMPP), or of d-*trans*-ChA•*1R*,*2R*-(-)-(DMPP) and I-*trans*•*1R,2R-*(-)-(DMPP) respectively.

It has also been observed that equal quantities of the *n* salt I-*trans*-ChA•*1S*,*2S*-(+)-(DMPP), and the *p* salt d-*trans*-ChA•*1S*,*2S*-(+)-(DMPP), when mixed in isopropyl ether at reflux and maintained at boiling point for 0.5 hour, provide with high yield a precipitate with the typical characteristics of the *p₁ n₁* salt dI-*trans*-ChA•*1S*,*2S-*(+)-(DMPP) which remains unchanged after repeated recrystallizations.

The present invention has enabled high yield d-*trans*-ChA to be obtained without recourse to methanol to promote nucleation and crystal growth. Not using methanol as co-solvent is favourable because of the reduced toxicity to man and the higher activity/weight ratio of the d-*trans*-ChA crystal obtained. Furthermore, the use of a single solvent (isopropyl ether) during both reactions with DMPP and

MTDP and during the various washes, substantially simplifies the preparation method and design of the production plants.

The effectiveness of the process does not diminish by operating in the presence of *cis*-ChA isomers and hence enables the results obtained by asymmetric catalysis, frequently characterised by insufficient diastereo- and enantioselectivity, to be improved, thus leading to enantiomerically pure *trans-*chrysanthemic acid.

DMPP has proved to be highly selective towards the *trans*-ChA diastereoisomer, allowing selective precipitation from isopropyl ether in salt form; the salification yield (calculated as percentage *trans* isomer obtained relative to the quantity of *trans* isomer present initially) is high, being in the order of 80-90%; the diastereoisomeric purity of the product is substantially equal to 100%. Likewise, the distinctive and exclusive property of DMPP enantiomers was discovered of generating *p₁,n₁* salts solely with trans-ChA isomers while not being reactive towards *cis*-ChA isomers. The aforesaid DMPP enantiomers are known and commercially available.

MTDP has proved to be highly enantioselective towards the d-*trans*-ChA enantiomer, the salification yield being in this case also high, in the order of 80-90%. The enantiomeric purity obtained is substantially equal to 100%.

Both *1R*,*2R*-(-)MTDP and *1S*,*2S*-(+)MTDP are new compounds and as such constitute a further aspect of the invention. They were prepared by N,N-bismethylation of *1R*,*2R*-(-)dimethylamino-1-phenyl-1,3-propanediol and its enantiomer respectively, well known as intermediates in the industrial synthesis of chloramphenicol, following the method of Clarke, H.T. et al., J.Am.Chem.Soc., 1933, 55, 4571 and the contribution regarding protocols made by Cope, A.C. et al J.Am.Chem.Soc., 1960, 82, 4651.

The use of DMPP and MTDP implies further industrial advantages in that both are N,N-dimethyl derivatives of easily accessible amines, produced industrially with high optical purity and able to be recovered safely and practically at the end of the process and recycled in later salification cycles without undergoing any loss of activity, with evident cost savings.

The invention is illustrated hereinafter by the following non-limiting examples.

### EXPERIMENTAL PART

Chrysanthemic acids and the bases utilized and obtained as products were characterised by means of GC, HPLC, TLC and IR, ¹H NMR, ¹³C NMR and mass spectrophotometric techniques.

The **[α]_{D}** values were measured at 20°C where not otherwise indicated.

The diastereoisomeric and enantiomeric composition of the various chrysanthemic acid samples was analysed by GC (capillary column Rt-βDEXsm™-RESTEK Corp.-30mt; 0.32 mmID; 0.25µmf; injection temperature 275°C; FID Detector at 300°C; programme: 80°C (2 min) then at 125°C (1.5°C/min)).

### 1. Physico-chemical and spectroscopic characteristics of the salts

### 1.1) dl-trans-ChA•1S,2S-(+)-DMPP (p₁,n₁ salt)

*[(1S,2S)-(+)-1,3-dihydroxy-N,N-dimethyl-1-phenylpropan-2-amino d,I-trans-2,2-dimethyl-3-(2-methylprop-1-en-1-yl) cyclopropanecarboxylafe]:*
**¹H-NMR** (300 MHz, CDCl₃): δ, ppm: 1.08 (*s*, 3H, CH₃(*acid*)); 1.22 (*s*, 3H, CH3(*acid*)); 1.30 (*dd,* 1H, J₁=5.40Hz, J₂=1.78Hz, CH(*acid*)); 1.68 (*s*, 6H, 2CH₃(*acid*)); 1.94 (*dd,* 1H, J₁=7.99Hz, J₂=5.40Hz, CH(*acid*)); 2.71 (*s*, 6H, 2CH₃, N-CH₃); 2.94 (*m*, 1H, CH(*amine*)); 3.33 (*dd*, 1Hₐ, J₁=12.93Hz, J₂=6.22Hz, CH₂(*amine*)); 3.52 (*dd*, 1H_{b}, J₁=12.93Hz, J₂=2.87Hz, CH₂(*amine*)); 4.67 (*d*, 1H, J₁=9.58Hz, CH(*amine*)); 4.85 (*d*, 1H, J₁=5.27Hz, CH(*acid*)); 7.34 (*m*, 5H, CH, Ar-H(*amine*)); 7.41 (s, 3H, 20H, NH).
**¹³C-NMR** (75 MHz, CDCl₃): δ, ppm: 19.10; 21.35; 23.06; 26.16 (CH₃(*acid*)); 27.94 (C(*acid*)); 32.31; 32.42; 37.49; 37.63 (CH(*acid*), (2 diastereoisomers)); 42.06 (N-CH₃); 58.41 (CH₂(*amine*)); 71.51; 71.69 (CH(*amine*)); 122.80 (CH(*acid*)); 122.85; 127.68; 128.91; 129.27(CH, Ar-CH); 134.85 (C(*acid*)); 141.86 (C; Ar-C); 179.60 (C=O).
**I.R.** ( KBr) ν: 3151 (br, OH); 2922 (NH); 1568 (s, COO⁻); 1421 (m, COO⁻) cm⁻¹
**Mass: (ES+):** 296(M⁺+1); 297(M⁺+2); **(ES-):**167(M⁻-1); 168(M⁻)
**[α]_{D}** = + 26.8° (c=1.036, CHCl₃); **mp:** 109.7-111.5°C

### 1.2) dI-trans-ChA·1R,2R-(-)-DMPP (p₁,n₁ salt)

### [(1R,2R)-(-)-1,3-dihydroxy-N,N-dimethyl-1-phenylpropan-2-amino d,I-trans-2, 2-dimethyl-3-(2-methylprop-1-en-1-yl)cyclopropanecarboxylate]

This salt is the levorotary enantiomer of the preceding *p₁,n₁* salt, the physico-chemical and spectroscopic characteristics of which are the same.

### 1.3) I-trans-ChA-1S,2S-(+)-DMPP (n salt)

### [(1S,2S)-(+)-1,3-dihydroxy-N,N-dimethyl-1-phenylpropan-2-amino (1S,3S)-(-)-2,2-dimethyl-3-(2-methylprop-1-en-1-yl)cyclopropanecarboxylate]

**¹H-NMR** (300 MHz, CDCl₃): δ, ppm: 1.08 (*s*, 3H, CH₃(*acid*)); 1.22 (*s*, 3H, CH₃(*acid*)); 1.30 (*dd,* 1H, J₁=5.40Hz, J₂=1.78Hz, CH(*acid*)); 1.69 (*s*, 6H, 2CH₃(*acid*)); 1.94 (*dd,* 1H, J₁=7.99Hz, J₂=5.40Hz, CH(*acid*)); 2.71 (*s*, 6H, 2CH₃, N-CH₃); 2.94 (*m*, 1H*, CH(amine*)); 3.31 (*dd*, 1Hₐ, J₁=12.93Hz, J₂=6.22Hz, CH₂(*amine*)); 3.54 (*dd*, 1H_{b}, J₁=12.93Hz, J₂=2.87Hz,CH₂(*amine*)); 4.68 (d, 1H, J₁=9.58Hz, CH(*amine*)); 4.85 (*d*, 1H, J₁=5.27Hz, CH(*acid*)); 7.32 (m, 5H, CH, Ar-H(*amine*)); 7.67 (s, 3H, 2OH, NH).
**¹³C-NMR** (75 MHz, CDCl₃ ): δ, ppm: 18.60; 20.83; 22.57; 25.67 (CH₃(*acid*)); 27.33 (C(*acid*)); 31.77; 37.56 (CH(*acid*)); 41.53 (N-CH₃); 57.83 (CH₂(*amine*)); 71.00 (CH(*amine*)); 122.37 (CH(*acid*)); 127.20, 128.42, 128.77 (CH, Ar-CH); 134.32 (C(*acid*)); 141.39 (C; Ar-C); 179.20 (C=O).
**I.R.** ( KBr) ν: 3151 (br, OH); 2922 (NH); 1568 (s, COO⁻); 1421 (m, COO⁻) cm⁻¹
**Mass: (ES+):** 296(M⁺+1); 297(M⁺+2); **(ES-)**:167(M⁻-1); 168(M⁻)
**[α]_{D}** = + 12.3° (c=0.9720, CHCl₃); **mp:** 132.6-134.0°C

### 1.4) d-trans-ChA•1R,2R-(-)-DMPP (n salt)

### [(1R,2R)-(-)-1,3-dihydroxy-N,N-dimethyl-1-phenylpropan-2-amino (1R,3R)-(+)-2,2-dimethyl-3-(2-methylprop-1-en-1-yl)cyclopropanecarboxylate]:

This salt is the levorotary enantiomer of the preceding n salt, the physico-chemical and spectroscopic characteristics of which are the same.

### 1.5) I-trans-ChA•1S,2S-(+)-MTDP (n salt)

### {(1S,2S)-(+)-1,3-dihydroxy-N,N-dimethyl-1-[4-(methylthio)phenyl]propan-2-amino (1S,3S)-(-)-2,2-dimethyl-3-(2-methylprop-1-en-1-yl)cyclopropanecarboxylate}:

**¹H-NMR** (300 MHz, CDCl₃): δ, ppm: 1.08 (*s*, 3H, CH₃(*acid*)); 1.22 (*s*, 3H, CH₃(*acid*)); 1.30 (*dd*, 1H, J₁=5.40Hz, J₂=1.78Hz, CH(*acid*)); 1.68 (*s*, 6H, 2CH₃(*acid*)); 1.94 (*dd*, 1H, J₁=7.99Hz, J₂=5.40Hz, CH(*acid*)); 2.44 (*s*, 3H, CH₃, S-CH₃); 2.73 (*s*, 6H, 2CH₃, N-CH₃); 2.92 (*m*, 1H, CH(*amine*)); 3.33 (*dd*, 1Hₐ, J₁=12.93Hz, J₂=6.22Hz, CH₂(*amine*)); 3.57 (*dd*, 1H_{b}, J₁=12.93Hz, J₂=2.87Hz, CH₂(*amine*)); 4.67 (*d*, 1H, J₁=9.58Hz, CH(*amine*)); 4.85 (*d*, 1H, J₁=5.27Hz, CH(*acid*)); 7.18 (m, 5H, CH, Ar-H(*amine*)); 7.28 (*d*, 1H, J₁=8.82Hz, CH, Ar-H); 7.36 (s, 3H, 2OH, NH).
**¹³C-NMR** (75 MHz, CDCl₃): δ, ppm: 19.10; 21.35; 23.06; 26.16 (CH₃(*acid*)); 27.94 (C(*acid*)); 32.31; 32.42; 37.49; 37.63 (CH(*acid*)); 42.06 (N-CH₃); 58.41 (CH₂(*amine*)); 71.51; 71.69 (CH(*amine*)); 122.80 (CH(*acid*)); 122.85; 127.68; 128.91; 129.27(CH, Ar-CH); 134.85 (C(*acid*)); 141.86 (C; Ar-C); 179.60 (C=O).
**I.R.** ( KBr) ν: 3254 (br, OH); 2915 (NH); 1576 (s, COO⁻); 1424 (m, COO⁻) cm⁻¹.
**Mass: (ES+):** 242(M⁺+1); 243(M⁺+2); (ES-): 167(M⁻-1); 168(M⁻)
**[α]_{D}** = +10.5° (c =0.986, CHCl₃); **mp:** 138-140°C

### 1.6) d-trans-ChA•1R,2R-(-)-MTDP (n salt)

### {(1R,2R)-(-)-1,3-dihydroxy-N,N-dimethyl-1-[4-(methylthio)phenyl]propan-2-amino (1R,3R)-(+)-2,2-dimethyl3-(2-methylprop-1-en-1-yl)cyclopropanecarboxylate}:

This salt is the levorotary enantiomer of the preceding n salt, the physico-chemical and spectroscopic characteristics of which are the same.

### Example 1

### Separation of d,I-trans-chrysanthemic acid from racemic chrysanthemic acid, with a trans:cis diastereoisomeric ratio of 65:35 using (1S, 2S)-(+)-DMPP.

(1*S*,2*S*)-(+)-2-dimethylamino-1-phenyl-1,3-propaediol [(1S,2S)-(+)-DMPP], 41.0 g (0.21 moles), is added to 200 ml of isopropyl ether. The mixture is heated under reflux (65°C) and to it a solution of 50.5 g (0.30 moles) racemic chrysanthemic acid, of *trans:cis* diastereoisomeric ratio 65:35, in 100 ml of isopropyl ether is slowly added.

At the end of the addition, precipitation of the corresponding salt is observed after a few minutes.

The mixture is maintained at 65°C under agitation for 45 minutes.

It is left to cool to ambient temperature and then to 10°C under agitation.

The salt is filtered and washed twice with 50 ml of isopropyl ether.

After drying, 71.9 g of the d-*p₁,n₁* salt dI-*trans*-ChA•1S,2S-(+)-DMPP are obtained. 500 ml of toluene are added to the salt obtained and the mixture is heated under reflux until the salt has dissolved.

The mixture is cooled to ambient temperature with precipitation of the salt and then it is cooled to 10°C.

The solid thus obtained is filtered off to obtain, after drying, 59.6 g of the pure d-*p₁*,*n₁* salt dI-*trans*-ChA•1S,2S-(+)-DMPP, with mp = 110-111.5°C; [α]_{D} = + 26.8° (c 1.036, chloroform).

The salt is dissolved in a 1 N aqueous HCl solution and extracted with toluene to provide, after evaporating under reduced pressure, 26.1 g of *trans*-chrysanthemic acid (total yield of 80.3%) with a *trans:cis* diastereoisomeric ratio of 99.5:0.5 ee (I-*trans*) = 2.6%.

In the mother liquors made alkaline (pH 10) with NaOH, precipitation of the base (1*S*,2*S*)-(+)-DMPP (30.4g, yield 75%) is observed, which is recovered optically intact by decanting, repeated washes with water and subsequent drying under vacuum. The residual mother liquors can be extracted with isopropyl ether (2 x 150 ml) to recover the residual quantity of base.

The mother liquors, made-alkaline (pH 10) with NaOH, give rise to the precipitation of 8.71 g of base found to be enantiomerically intact.

### Example 2

### Separation of d,I-trans-chrysanthemic acid from racemic chrysanthemic acid with a trans:cis diastereoisomeric ratio of 65:35 using (1R, 2R)-(-)-DMPP.

Separation of the dI*-trans* isomer from the dl-cis isomer of chrysanthemic acid was carried out by following the same procedure described in example 1 but using the base (1*R*,2*R*)-(-)-2-dimethylamino-1-phenyl-1,3-propanediol, [(1*R*,2*R*)-(-)-DMPP]. Formation of the I-*p₁*,*n₁* salt i.e. dI-*trans*-ChA•1*R*,2*R*-(-)-DMPP is thus induced:
mp = 110-111.5°C;
[α]_{D} = -26.4° (c 1.032, chloroform), enantiomer of the d- p₁,*n*₁ salt.

### Example 3

### Separation of d,I-trans-chrysanthemic acid from racemic chrysanthemic acid with a trans:cis diastereoisomeric ratio of 80:20 using (1S, 2S)-(+)-DMPP.

Similarly to what is described in example 1, 46.9 g (0.24 moles) of (1*S*,2*S*)-(+)-2-dimethylamino-1-phenyl-1,3-propanediol [(1*S*,2*S*)-(+)-DMPP] in 250 ml of isopropyl ether are reacted with 50 g (0.30 moles) of racemic chrysanthemic acid with *trans:cis* diastereoisomeric ratio of 80:20 dissolved in 50 ml of isopropyl ether. A while after the addition precipitation of the corresponding salt is noted after a while.

The mixture is maintained at 65°C under agitation for 1.5 hours.

It is cooled to ambient temperature and then cooled to 10°C.

The salt is filtered and washed twice with 50 ml of isopropyl ether.

After drying 81.7 g of salt are obtained.

Analysis (GC on chiral column) of a salt sample, after treating with 1 N HCl and extracting with ethyl ether, indicates a chrysanthemic acid with a *trans:cis* diastereoisomeric ratio of 95:5, an ee (I-*trans*) = 0.6% and an ee = 0%. The salt thus obtained is treated as described in example 1, to obtain after drying, 77.8 g of the d-*p₁*,*n₁* salt dI-*trans*-ChA•1*S*,2*S*-(+)-DMPP, pure: mp = 110-111.5°C; [α]_{D} = + 26.8° (c 1.036, chloroform).

The salt thus obtained, after treating as described in example 1, provided 36.0 g of trans-chrysanthemic acid (100% *trans*, ee (I-*trans*) = 2.6%) with quantitative yield.

Recovery of the enantiomerically intact base (1*S*,2*S*)-(+)-DMPP was achieved as described in example 1.

### Example 4

### Separation of d,I-trans-chrysanthemic acid from racemic chrysanthemic acid with a trans:cis diastereoisomeric ratio of 80:20 using (1R, 2R)-(-)-DMPP.

Separation of the dl-trans isomer from the dI-*cis* isomer of chrysanthemic acid is carried out by following the same procedure described in example 1 but using the base (1*R*,2*R*)-(-)-2- dimethylamino-1-phenyl-1,3-propanediol, [(1*R*,2*R*)-(-)-DMPP].

Formation of the I-*p₁*,*n₁* salt i.e. dI-*trans*-ChA•1*R*,2*R*-(-)-DMPP is thus induced: mp = 110-111.5°C;
[α]_{D} = -26.4° (c 1.032, chloroform), enantiomer of the d-*p₁*,*n₁* salt

### Example 5

### Resolution of racemic trans-chrysanthemic acid by the formation of n salts with the enantiomers (1R, 2R)-(-)-MTDP and (1S,2S)-(+)-MTDP.

480 ml of isopropyl ether are added to 67.6 g (0.28 moles) of (1 R,2R)-(-)-2-dimethylamino-1-[4-(methylthio)phenyl]-1,3-propanediol [(1*R*,2*R*)-(-)MTDP]. The mixture is heated under reflux until completely dissolved then a solution of 90.8 g (0.54 moles) of racemic *trans*-chrysanthemic acid is added, prepared as described in examples 1 and 2, in 220 ml of isopropyl ether.

After a while precipitation of the salt is observed. The mixture is left under reflux and stirred for 30 minutes. It is cooled to ambient temperature and the precipitated salt is separated by filtration. The salt is then washed three times with 100 ml of isopropyl ether and dried at 25°C/24 mbar to obtain 90.8 g of the crude I-*n* salt, d-*trans*-ChA•1*R*,2*R*-(-)-MTDP. GC analysis of a sample after the usual displacement of the base shows d-*trans*-chrysanthemic acid with an ee = 94%. 150 ml of isopropyl ether are added to the salt thus obtained and the mixture is heated at reflux for 30 minutes while being stirred.

The mixture is cooled to ambient temperature and the salt separated by filtration then dried under vacuum at 30°C/24 mbar to obtain 88.4 g of pure I-*n* salt: mp = 138-140°C;
[α]_{D} = - 10.5° (c 0.986, chloroform).

The salt is dissolved in a 1 N aqueous HCl solution and extracted with toluene to provide, after evaporation under reduced pressure, 36.3 g (total yield 80%) of d-*trans*-chrysanthemic acid, d-*trans-*ChA (ee = 97%).

The base (1R,2*R*)-(-)-MTDP, used as resolving agent, precipitates from the mother liquors made alkaline with NaOH. It is recovered (51.7 g, yield 76.2%), optically intact, by filtering, frequent washing with water and subsequent drying under vacuum. The remaining mother liquors can be extracted with isopropyl ether (2 x 100 ml) to recover the remaining quantity of base (5.4 g).

The filtered isopropyl ether solution, to which the isopropyl ether used for washing the I-*n* salt is added, is washed with water acidified (pH 3.5) with 1 N hydrochloric acid, then washed several times with water, dried over MgSO₄ and evaporated under reduced pressure to provide 52.14 g of I-*trans* chrysanthemic acid (ee = 73%). 10.22 g of basic resolving agent precipitate from the mother liquors alkalinized to pH 10 with NaOH for an overall recovery of 99% of the base (1*R*,2*R*)-(-)-2-dimethylamino-1-[4-(methylthio)phenyl]-1,3-propanediol.

The I-*trans* chrysanthemic acid thus obtained (52.1 g, ee = 73%, 0.31 moles) is dissolved in 420 ml of isopropyl ether, brought to boiling point and 64.3 g (0.27 moles) of (1S,2S)-(+)-2-dimethylamino-1-[4-(methylthio)phenyl]-1,3-propanediol [(1S,2S)-(+)MTDP] are added. The reaction mixture is kept boiling for ½ hour then left to cool to ambient temperature. The precipitated d-n salt (I-*trans*-ChA·1S,2S-(+)-MTDP) (102.0 g), an enantiomer of the previous salt, is collected by filtration: mp 138-140°C, [α]_{D} = +10.5° (c 1.154, chloroform) and provides, when displaced with acid and extracted with toluene in the usual manner, 41.23 g (total yield 90.73%) of I-*trans* chrysanthemic acid (ee = 98%) by evaporation under reduced pressure. Alkalinization of the mother liquors with NaOH as aforedescribed enables precipitation of 59.12 g of the base (1*S*,2*S*)-(+)-MTDP used as resolving agent. Subsequent extraction of the mother liquors with ether enables the total recovery of the base, shown to be enantiomerically intact. A residue remains in the mother liquors of salt precipitation, which, when suitably treated, provides 13.56 g of d-*trans*-chrysanthemic acid (ee = 47%).

### Example 6

### Recovery of the enantiomeric excess of d-trans-ChA from a scalemic mixture with ee = 60% via d-p₁,n₁ salt formation and use of (1S,2S)-(+)-DMPP.

*(1S,2S)-(+)-DMPP* (2.34 g, 0.012 moles) is dissolved under hot conditions in 40 ml of isopropyl ether and to this solution is added 5.0 g (0.03 moles) of a scalemic mixture of d-*trans*/I-*trans* chrysanthemic acid in a 4:1 ratio (e.e. = 60%) in 40 ml of isopropyl ether. The mixture is heated at 65°C for 30 minutes while being stirred and then cooled to ambient temperature. The precipitation of a solid is observed. It is cooled to 0°C, stirred for 15 minutes, the solid obtained is filtered off and washed with two (9 ml) portions of isopropyl ether.

The solid is dried and provides 2.34 g of d-*p₁*,*n₁* salt i.e. dI-*trans*-ChA•1*S*,2*S*-(+)-DMPP: mp 110-111.5°C; [α]_{D} = +26.8° (c 1.036, chloroform). The displacement of the salt components, undertaken as described in the preceding examples, provides dl-*trans*-ChA and enables the base to be recovered.

The filtered ether solution, to which the washing waters are added, is washed with water acidulated to pH 3.5, dried and evaporated at 30°C/24 mbar, to provide 3.1 g of d-*trans*-chrysanthemic acid (ee >95%).

### Example 7

### Recovery of the enantiomeric excess of I-trans-ChA from a scalemic mixture with ee = 60% via I-p₁,n₁ salt formation and use of (1R,2R)-(-)-DMPP.

A scalemic mixture of I-*trans*/d-*trans*-chrysanthemic acid (5.0 g, 0.03 moles) in a 4:1 ratio (e.e. = 60%) was treated with (1*R*,2*R*)-(-)-DMPP (2.34 g, 0.012 moles) as described in example 4 to provide 3.27 g of I-*p*₁,*n*₁ salt i.e. dI-*trans*-ChA•1*R*,2*R*-(-)-DMPP: mp 110-111.5°C; [α]_{D} = - 26.3° ( c 1.026, chloroform). Displacement of the salt components undertaken as described in the preceding examples provides dI-*trans*-ChA and enables the base to be recovered.

The filtered ether solution, to which washing waters are added, is washed with water acidulated to pH 3.5, dried and evaporated at 30°C/24 mbar, to provide 3.0 g of I-*trans* chrysanthemic acid (ee >95%).

### Example 8

### Recovery of the enantiomeric excess of d-trans-ChA from a scalemic mixture with ee = 80% via d-p₁,n₁ salt formation and use of (1S,2S)-(+)-DMPP.

Similarly to what is described in example 4, 1.17 g (0.006 moles) of (1*S*,2*S*)-(+)-DMPP dissolved in 35 ml of isopropyl ether are reacted with 5.0 g (0.03 moles) of d-*trans*/I*-trans* chrysanthemic acid with a 90:10 ratio (e.e. = 80%), dissolved in 20 ml of isopropyl ether.

After treatment analogous to that described in example 4, 1.89 g of d-*p₁*,*n₁* i.e. dI-*trans*-ChA•1*S*,2*S*-(+)-DMPP are obtained: mp 110-111.5°C; [α]_{D} = + 26.8° (c 1.036, chloroform). Displacement of the salt components undertaken as described in the preceding examples provides dI-*trans*-ChA and allows the base to be recovered.

The filtered ether solution, to which washing waters are added, is washed with water acidulated to pH 3.5, dried and evaporated at 30°C/24 mbar, to provide 3.98 g of d-*trans*-chrysanthemic acid (ee >95%).

### Example 9

### Recovery of the enantiomeric excess of I-trans-ChA from a scalemic mixture with ee = 80% via I-p₁,n₁ salt formation and use of (1R,2R)-(-)-DMPP.

A scalemic mixture of I-*trans*/d-*trans*-chrysanthemic acid (5.0 g, 0.03 moles) in a 90:10 ratio (ee = 80%) was treated with (1*R*,2*R*)-(-)-DMPP (1.16 g, 0.006 moles) as described in example 4 to provide 1.95 g of I-*p₁*,*n₁* salt i.e. dI-*trans*-ChA·1*R,*2*R-*(-)-DMPP: mp 110-111.5°C;
[α]_{D} = - 26.4° (c 1.029, chloroform). The displacement of the salt components, undertaken as described in the preceding examples, provides dI-*trans*-ChA and enables the base to be recovered.

The filtered ether solution, to which washing waters are added, is washed with water acidulated to pH 3.5, dried and evaporated at 30°C/24 mbar, to provide 4.0 g of I-*trans* chrysanthemic acid (ee >95%).

### Example 10

### Recovery of the enantiomeric excess of d-trans-ChA from a scalemic mixture with ee = 60% via I-n salt formation and use of (1R,2R)-(-)-DMPP.

*(1R,2R)-(-)-DMPP* (3.51 g, 0.018 moles) is dissolved under hot conditions in 40 ml of isopropyl ether. Added to this solution are 5.0 g (0.03 moles) of a scalemic mixture of d-*trans*/I*-trans* chrysanthemic acid in a 4:1 ratio (ee = 60%) in 40 ml of isopropyl ether. The mixture is heated to 65°C for 30 minutes while being stirred and then cooled to ambient temperature. The precipitation of a solid is observed. It is cooled to 0°C. stirred for 15 minutes, the solid obtained is filtered off and washed with two (9 ml) portions of isopropyl ether.

The solid is dried and provides 6.43 g of I-*n* salt i.e. d-*trans*-ChA•1R,2R-(-)-DMPP: mp 135-137°C; [α]_{D} = -12.3° (c 0.9720, chloroform). The displacement of the salt components, undertaken as described in the preceding examples, provides 2.90 g of d-*trans*-ChA (ee > 95%) and allows the base to be recovered.

The filtered ether solution, to which washing waters are added, is washed with water acidulated to pH 3.5, dried and evaporated at 30°C/24 mbar, to provide 1.98 g of dl-trans chrysanthemic acid.

### Example 11

### Recovery of the enantiomeric excess of I-trans-ChA from a scalemic mixture with ee = 60% via d-n salt formation and use of (1S,2S)-(+)-DMPP.

A scalemic mixture of I-*trans*/d-*trans*-chrysanthemic acid (5.0 g, 0.03 moles) in a 4:1 ratio (e.e. = 80%) was treated with (1S,2S)-(+)-DMPP (4.69 g, 0.024 moles) as described in example 6 to provide 6.47 g of d-n salt i.e. I-*trans*-ChA•1S,2S-(+)-DMPP: mp 133-135°C; [α]_{D} = +12.2° (c 1.021, chloroform). Displacement of the salt components undertaken as described in the preceding examples provides 2.87 g of I-*trans*-ChA (ee >95%) and enables the base to be recovered.

The filtered ether solution, to which washing waters are added, is washed with water acidulated to pH 3.5, dried and evaporated at 30°C/24 mbar, to provide 1.92 g of dI-*trans* chrysanthemic acid.

### Example 12

### Recovery of the enantiomeric excess of d-trans-ChA from a scalemic mixture with ee = 80% via I-n salt formation and use of (1R,2R)-(-)-DMPP.

*(1R,2R)-(-)-DMPP* (4.68 g, 0.024 moles) is dissolved under hot conditions in 40 ml of isopropyl ether. Added to this solution are 5.0 g (0.03 moles) of a scalemic mixture of d-*trans*/I-*trans* chrysanthemic acid in a 9:1 ratio (ee = 80%) in 40 ml of isopropyl ether. The mixture was treated as described in example 6.

The solid obtained by filtration is dried and provides 8.58 g of I-*n* salt i.e. d-*trans-*ChA•1R,2R-(-)-DMPP: mp 133-135°C; [α]_{D} = -12.1° (c 0.9875, chloroform). Displacement of the salt components undertaken as described in the preceding examples provides 3.85 g of d-*trans-*ChA (ee > 95%) and allows the base to be recovered.

The filtered ether solution, to which washing waters are added, is washed with water acidulated to pH 3.5, dried and evaporated at 30°C/24 mbar, to provide 0.95 g of dl-trans chrysanthemic acid.

### Example 13

### Recovery of the enantiomeric excess of I-trans-ChA from a scalemic mixture with ee = 80% via d-n salt formation and use of (1S,2S)-(+)-DMPP.

A scalemic mixture of I-*trans*/d-*trans*-chrysanthemic acid (5.0 g, 0.03 moles) in a 9:1 ratio (ee = 80%) was treated with (1S,2S)-(+)-DMPP (4.68 g, 0.024 moles) as described in example 7 to provide 8.63 g of d-*n* salt i.e. I-*trans*-ChA•1S,2S-(+)-DMPP: mp 133-135°C; [α]_{D} = +12.2° ( c 1.021, chloroform). Displacement of the salt component undertaken as described in the preceding examples provides 3.92 g of I-*trans*-ChA (ee >95%) and allows the base to be recovered.

The filtered ether solution, to which washing waters are added, is washed with water acidulated to pH 3.5, dried and evaporated at 30°C/24 mbar, to provide 0.91 g of dl-trans chrysanthemic acid.

### Example 14

### Recovery of the enantiomeric excess of d-trans-ChA from a scalemic mixture with ee = 80% via I-n salt formation and use of (1R,2R)-(-)-DMPP in the presence of dI-cis-ChA (20%).

*(1R,2R)-(-)-DMPP* (12.5 g, 0.064 moles) is dissolved under hot conditions in 80 ml of isopropyl ether. Added to this solution are 16.8 g (0.1 moles) of chrysanthemic acid, composed of 20% dI-*cis*-ChA (0.02 moles) and 80% *trans*-ChA (0.08 mol, ee 80%: d-*trans* 0.072 mol; I-*trans* 0.008 moles), in 40 ml of isopropyl ether. The mixture was treated as described in example 6.

The solid obtained by filtration is dried to provide 22.8 g of I-*n* salt i.e. d-*trans-*ChA•1R,2R-(-)-DMPP: mp 133-135°C; [α]_{D} = -12.1° (c 0.9875, chloroform). Displacement of the salt components undertaken as described in the preceding examples provides 9.8 g of d-*trans*-ChA (ee > 95%) and allows the base to be recovered.

The filtered ether solution, to which washing waters are added, is washed with water acidulated to pH 3.5, dried and evaporated at 30°C/24 mbar, to provide 5.90 g of chrysanthemic acid consisting of dI-*cis* and I-*trans*.

### Example 15

*Recovery of the enantiomeric excess of d-trans-ChA from a scalemic mixture with ee = 80% in the presence of dI-cis-ChA (20%) via d-p₁,n₁ salt formation with use of (1S,2S)-(+)-DMPP, and I-n salt formation with use of (1R,2R)-(-)-DMPP.*

*(1S,2S)-(+)-DMPP* (3.12 g, 0.016 moles) is dissolved under hot conditions in 80 ml of isopropyl ether. Added to this solution are 16.8 g (0.1 moles) of chrysanthemic acid composed of 20% dl-*cis*-ChA (0.02 moles) and 80% *trans*-ChA (0.08 mol, ee 80%: d-*trans* 0.072 mol; I-*trans* 0.008 moles) in 40 ml of isopropyl ether. The mixture was treated as described in example 6.

The solid obtained by filtration is dried and provides 5.72 g of d-*p₁*,*n₁* i.e. *dl-trans-*ChA•1S,2S-(+)-DMPP: mp 110-111.5°C; [α]_{D} = + 26.6° (c 1.038, chloroform).

The filtered ether solution, washed with water acidulated to pH 3.5, dried and evaporated at 30°C/24 mbar, provides 10.5 g of chrysanthemic acid consisting of (GC) dl-cis and excess d-*trans.* Separation of the d-*trans*-ChA acid from the *cis-*ChA isomers is achieved by dissolving the mixture in isopropyl ether (30 ml) and adding a solution of (1*R*,2*R*)-(-)-DMPP dissolved under hot conditions in 40 ml of isopropyl ether. The mixture is treated as described in example 6. The solid obtained by filtration is dried and provides 22.81 g of I-*n* salt, i.e. *d-trans-*ChA•1*R*,2*R*-(-)-DMPP : mp 133-135°C; [α]_{D}= -12.1° (c 0.9875, chloroform).

Displacement of the d-*p₁*,*n₁* salt and I-*n* salt undertaken as described in the preceding examples provides 2.55 g of dI-*trans*-ChA and 10.3 g *d-trans-ChA* respectively (ee >95%) with recovery of the respective bases.

Evaporation of the residual ether solution provides 3.28 g of dI-*cis*-ChA.

### Example 16

### Synthesis of (1S,2S)-(+)-2-dimethylamino-1-[4-(methylthio)phenyl]-1,3-propanediol [(1S,2S)-(+)MTDP].

(1*S*,2*S*)-(+)-2-amino-1-[4-(methylthio)phenyl]-1,3-propanediol (42.7 g, 0.2 moles) was added in small portions at 0°C to 32.2 g (0.7 moles) of formic acid, followed by the addition of 43 ml (0.05 moles) of a 35% aqueous solution of formaldehyde.

The solution was then heated to 90°C for 24 hours. After leaving to cool, 17 ml of 6N HC! were added and the mixture extracted with dichloromethane (3 x 20ml). A 20% aqueous NaOH solution was then added to the thus purified aqueous phase until the pH was basic. The alkaline waters are extracted -with dichloromethane. The organic solution is dried over MgSO₄, filtered off and evaporated under reduced pressure to provide 41.40 g (yield 86%) of *N*,*N*-dimethyl derivative: mp 89-91.5 °C; [α]¹⁹_{D} = +24.0° (c 1.52, acetone); [α]¹⁹_{D} = +39.9° (c 1.36, chloroform) . IR (KBr) δ: 3437; 2942; 1598; 1457 cm⁻¹. ¹H NMR (200 MHz, CDCl₃) δ: 2.45 (s, 9H); 2.60 (m, 1H); 3.38 (m, 2H); 4.30 (d, 1H, *J* = 9.6 Hz), 7.22 (m, 4H) ppm. ¹³C NMR (50 MHz, CDCl₃) δ: 16.42; 41.93; 58.74; 71.40; 71.70; 127.18; 128.16; 138.64; 139.31 ppm. Mass: 241, 240, 195, 154, 137.

### Example 17

### Synthesis of (1R,2R)-(-)-2-dimethylamino-1-[4-(methylthio)phenyl]-1,3-propanediol [(1R,2R)-(-)MTDP]

(1*R*,2*R*)-(-)-2-amino-1-[4-(methylthio)phenyl]-1,3-propanediol (42.0 g, 0.197 moles), 31.7 g (0.69 moles) of formic acid and 42.4 ml (0.49 moles) of a 35% aqueous formaldehyde solution were treated as described in example 15 to provide 47.5 g of *N*,*N*-dimethyl derivative (yield 88%): mp 89-91.5°; [α]¹⁹_{D} = -22.7° (c 1.49, acetone); [α]¹⁹_{D} = -37.6° (c 1.30, chloroform). IR (KBr), ¹H NMR, ¹³C NMR and Mass spectroscopic data proved to be identical to those found for (1*S*,2*S*)-(+)MTDP.

## Claims

1. Process for isolating d-*trans*-chrysanthemic acid (d-*trans*-ChA) and/or I-*trans* chrysanthemic acid (I-*trans*-ChA) starting from mixtures of isomers of said acid (ChA), comprising the use of enantiomers of 2-dimethylamino-1-phenyl-1,3-propanediol (DMPP) and 2-dimethylamino-1-[4-(methylthio)phenyl]propane-1,3-diol (MTDP) as chiral selectors.

2. Process as claimed in claim 1 for separating *cis*-ChA from *trans*-ChA, comprising the reaction of ChA with *1S*,*2S*-(+)-DMPP or with *1R,2R*-(-)-DMPP, utilized in amount of 1 equivalent relative to the quantity of *trans*-ChA present in said ChA.

3. Process as claimed in claim 1 for separating a racemic mixture of *trans*-ChA into its *d-trans* and I-*trans* enantiomers, comprising the reaction of said mixture with *1S*,*2S*-(+)-MTDP or *1R*,*2R*-(-)-MTDP in amount of 0.5 equivalents relative to the *trans*-ChA present in the mixture.

4. Process as claimed in claim 1 for separating a scalemic mixture of *trans*-ChA, comprising the reaction of said mixture with *1S*,*2S*-(+)-DMPP or with 1*R*,2*R*-(-)-DMPP.

5. Process as claimed in claim 4, wherein said scalemic mixture is enantiomerically enriched in d-, and where *1S*,*2S*-(+)-DMPP is utilised in amount of 2 equivalents relative to the lesser I- enantiomer, or *1R*,*2R*-(-)-DMPP is utilized in amount of 1 equivalent relative to the excess of d- enantiomer.

6. Process as claimed in claim 4, wherein said scalemic mixture is enantiomerically enriched in I-, and where *1S*,*2S*-(+)-DMPP is utilized in amount of 1 equivalent relative to the excess of I- enantiomer, or *1R*,*2R*-(-)-DMPP is utilized in amount of 2 equivalents relative to the lesser d- enantiomer.

7. Process as claimed in claims 1-6, wherein the reaction product with DMPP and MTDP is a salt of *p₁n₁* type or *n* type.

8. Process as claimed in claim 7, wherein the required enantiomer of *trans*-ChA is recovered from said *p₁n₁* salt by dissolving the salt in acid solution, extracting with organic solvent, recovering and evaporating the organic phase, dissolving the solid resulting from the evaporation in a suitable solvent and reacting the resulting solution with *1S*,*2S*-(+)-MTDP or *1R*,*2R*-(-)-MTDP in amount of 0.5 equivalents relative to the *trans*-ChA present.

9. Process as claimed in claim 7, wherein the required enantiomer of *trans*-ChA is recovered from said *n* salt by dissolving the salt in acid solution, extracting with organic solvent, recovering and evaporating the organic phase.

10. Process as claimed in claim 7 or 8, wherein the aqueous solution remaining after acidification and extraction is basified, recovering the DMPP or MTDP present in solution as precipitate in an enantiomerically pure form, with possibility of reuse in later salification cycles.

11. Process as claimed in claims 1-10, wherein the mother liquors obtained from reactions with DMPP or MTDP are further treated to recover the required enantiomers from the ChA remaining in solution.

12. Process as claimed in claim 10, wherein said mother liquors contain the diastereoisomer *cis*-ChA and where the required enantiomer (d-*trans* or I-*trans*) is obtained by converting *cis*-ChA into *trans*-ChA by treating with Lewis acids, and treating the resulting *trans*-ChA by means of the procedure described in claims 3-9.

13. Process as claimed in claim 10, wherein said mother liquors contain the diastereoisomer *trans*-ChA and where the required isomer (d*-trans* or I-*trans*) is recovered following the procedure described in claims 3-9.

14. Process as claimed in claim 10, wherein said mother liquors contain the d-*trans*-ChA enantiomer or the I-*trans* enantiomer, said d- or I- enantiomer being recovered by evaporating the respective mother liquors to dryness.

15. Process as claimed in claims 1-14, wherein the mixture of ChA isomers to be separated is **characterised by** a *trans*-ChA/*cis*-ChA ratio between 30/70 and 99/1.

16. Process as claimed in claims 1-14, wherein the mixture of ChA isomers to be separated is **characterised by** a trans-ChA/cis-ChA ratio chosen from 65/35, 80/20 or 92/8.

17. Process as claimed in claim 2, wherein all the salification reactions with DMPP and MTDP are undertaken in isopropyl ether.

18. A salt of chrysanthemic acid with *1S*,*2S*-(+)- or *1R*,*2R*-(-)-DMPP, chosen from:
dI-*trans*-ChA•*1S*,*2S*-(+)-2-dimethylamino-1-phenyl-1,3-propanediol;
dI-*trans*-ChA•*1R*,*2R*-(-)-2-dimethylamino-1-phenyl-1,3-propanediol;
I-*trans*-ChA•*1S*,*2S*-(+)-2-dimethylamino-1-phenyl-1,3-propanediol;
d-*trans*-ChA•*1R*,2*R*-(-)-2-dimethylamino-1-phenyl-1,3-propanediol;
d-*trans*-ChA•*1S*,*2S*-(+)-2-dimethylamino-1-phenyl-1,3-propanediol;
I-*trans*-ChA•*1R*,*2R*-(-)-2-dimethylamino-1-phenyl-1,3-propanediol;

19. A salt of chrysanthemic acid with *1S*,*2S*-(+)- or *1R*,*2R*-(-)-MTDP, chosen from:
I-*trans*-ChA•*1S*,*2S*-(+)-2-dimethylamino-1-[4-(methylthio)phenyl]propane-1,3-diol;
d-*trans*-ChA•*1R*,*2R*-(-)-MTDP-2-dimethylamino-1-[4-(methylthio)phenyl]propane-1,3-diol.

20. 2-dimethylamino-1-[4-(methylthio)phenyl]propane-1,3-diol.

## Patentansprüche

1. Verfahren zum Isolieren von d-trans-Chrysanthemensäure (d-trans-ChA) und/oder 1-trans-Chrysanthemensäure (1-trans-ChA, ausgehend von Mischungen von Isomeren der Säure (ChA), umfassend die Verwendung von Enantiomeren von 2-Dimethylamino-1-phenyl-1,3-propandiol (DMPP) und 2-Dimethylamino-1-[4-(methylthio-)phenyl-]propan-1,3-diol (MTDP) als chirale Selektoren.

2. Verfahren wie in Anspruch 1 beansprucht, zur Trennung von cis-ChA von trans-ChA, umfassend die Reaktion von ChA mit 1S,2S-(+)-DMPP oder mit 1R,2R-(-)-DMPP, verwendet in einer Menge von einem Äq/uivalent, bezogen auf die Menge von trans-ChA, die in dem ChA zugegen ist.

3. Verfahren wie in Anspruch 1 beansprucht, zur Trennung einer racemischen Mischung von trans-ChA in seine d-trans- und 1-trans-Enantiomere, umfassend die Reaktion der Mischung mit 1S,2S-(+)-MTDP oder 1R,2R-(-)-MTDP in einer Menge von 0,5 Äquivalenten, bezogen auf das trans-ChA, das in der Mischung zugegen ist.

4. Verfahren wie in Anspruch 1 beansprucht, zur Trennung einer nichtracemischen Mischung von trans-ChA, umfassend die Reaktion der Mischung mit 1S,2S-(+)-DMPP oder 1R,2R-(-)-DMPP.

5. Verfahren wie in Anspruch 4 beansprucht, worin die nicht-racemische (skalemische) Mischung Enantiomeren-mäßig angereichert an d-Enantiomer ist, und worin 1S,2S-(+)-DMPP in einer Menge von 2 Äquivalenten verwendet wird, bezogen auf das in geringerer Menge zugegene 1-Enantiomer, oder 1R,2R-(-)-DMPP verwendet wird in einer Menge von 1 Äquivalent, bezogen auf den Überschuss an d-Enantiomer.

6. Verfahren wie in Anspruch 4 beansprucht, worin die nicht-racemische (skalemische) Mischung Enantiomeren-mäßig angereicht an 1-Enantiomer ist, und worin 1S,2S-(+)-DMPP verwendet wird in einer Menge von 1 Äquivalent, bezogen auf den Überschuss an 1-Enantiomer, oder 1R,2R-(-)-DMPP verwendet wird in einer Menge von 2 Äquivalenten, bezogen auf das in geringerer Menge zugegene d-Enantiomer.

7. Verfahren wie in den Ansprüchen 1 bis 6 beansprucht, worin das Reaktionsprodukt mit DMPP und MTDP ein Salz des p₁n₁-Typs oder des n-Typs ist.

8. Verfahren wie in Anspruch 7 beansprucht, worin das erforderliche Enantiomer von trans-ChA aus dem p₁n₁-Salz gewonnen wird durch Lösen das Salzes in saurer Lösung, Extrahieren mit einem organischen Lösungsmittel, Gewinnen und Verdampfen der organischen Phase, Lösen des Feststoffs, der resultiert aus der Verdampfung in einem geeigneten Lösungsmittel und Umsetzen der resultierenden Lösung mit 1S,2S-(+)-MTDP oder 1R,2R-(-)-MTDP in einer Menge von 0,5 Äquivalenten, bezogen auf das zugegene trans-ChA.

9. Verfahren wie in Anspruch 7 beansprucht, worin das benötigte Enantiomer von trans-ChA aus dem n-Salz gewonnen wird durch Lösen des Salzes in saurer Lösung, Extrahieren mit einem organischen Lösungsmittel, Gewinnen und Verdampfen der organischen Phase.

10. Verfahren wie in Anspruch 7 oder 8 beansprucht, worin die wässrige Lösung, die nach dem Ansäuern und Extrahieren verbleibt, basisch gemacht wird, wodurch man das DMPP oder MTDP, das/die in Lösung zugegen ist/sind, als Niederschlag in einer Enantiomer-reinen Form gewinnt, mit der Möglichkeit, einer erneuten Verwendung in späteren Salzbildungszyklen.

11. Verfahren wie in den Ansprüchen 1 bis 10 beansprucht, worin die Mutterlaugen, die aus Reaktionen mit DMPP oder MTDP erhalten werden, weiter behandelt werden und so die erforderlichen Enantiomeren von der ChA, die in Lösung bleibt, gewonnen werden.

12. Verfahren wie in Anspruch 10 beansprucht, worin die Mutterlaugen das Diastereoisomer cis-ChA enthalten, und worin das erforderliche Enantiomer (d-trans oder 1-trans) erhalten wird durch Umwandeln von cis-ChA in trans-ChA durch Behandeln mit Lewis-Säuren und Behandeln der resultierenden trans-ChA mittels der Verfahrensweise, die in den Ansprüche 3 bis 9 beschrieben ist.

13. Verfahren wie in Anspruch 10 beansprucht, worin die Mutterlaugen das Diastereoisomer trans-ChA enthalten und worin das erforderliche Isomer (d-trans oder 1-trans) gewonnen wird, indem man der Verfahrensweise folgt, die in den Patentansprüchen 3 bis 9 beschrieben ist.

14. Verfahren wie in Anspruch 10 beansprucht, worin die Mutterlaugen das d-trans-ChA-Enantiomer oder das 1-trans-Enantiomer enthalten, wobei das d- oder 1-Enantiomer gewonnen wird durch Verdampfen der jeweiligen Mutterlaugen zur Trockene.

15. Verfahren wie in den Ansprüchen 1 bis 14 beansprucht, worin die Mischung von ChA-Isomeren, die getrennt werden sollen, **gekennzeichnet ist durch** ein Verhältnis trans-ChA/cis-ChA zwischen 30/70 und 99/1.

16. Verfahren wie in den Ansprüchen 1 bis 14 beansprucht, worin die Mischung von ChA-Isomeren, die getrennt werden sollen, **gekennzeichnet ist durch** ein Verhältnis trans-ChA/cis-ChA, das gewählt ist aus 65/35, 80/20 oder 92/8.

17. Verfahren wie in Anspruch 2 beansprucht, worin alle Salzbildungsreaktionen mit DMPP und MTDP in Isopropylether unternommen werden.

18. Salz von Chrysanthemensäure mit 1S,2S-(+)- oder 1R,2R-(-)-DMPP, gewählt aus:
dl-trans-ChA•1S,2S-(+)-2-Dimethylamino-1-phenyl-1,3-propandiol;
dl-trans-ChA•1R,2R-(-)-2-Dimethylamino-1-phenyl-1,3-propandiol;
l-trans-ChA•1S,2S-(+)-2-Dimethylamino-1-phenyl-1,3-propandiol;
d-trans-ChA•1R,2R-(-)-2-Dimethylamino-1-phenyl-1,3-propandiol;
d-trans-ChA•1S,2S-(+)-2-Dimethylamino-1-phenyl-1,3-propandiol;
l-trans-ChA•1R,2R-(-)-2-Dimethylamino-1-phenyl-1,3-propandiol.

19. Salz von Chrysanthemensäure mit 1S,2S-(+)- oder 1R,2R-(-)-MTDP, gewählt aus:
l-trans-ChA•1S,2S-(+)-2-Dimethylamino-1-[4-(methylthio-)phenyl-]propan-1,3-diol, d-trans-ChA•1,2R-(-)-MTDP-2-Dimethylamino-1-[4-(methylthio-)phenyl-]propan-1,3-diol.

20. 2-Dimethylamino-1-[4-(methylthio-)phenyl-]propan-1,3-diol

## Revendications

1. Procédé d'isolement d'acide d-*trans*-chrysanthémique (d-*trans*-ChA) et/ou d'acide 1-*trans*-chrysanthémique (1-*trans-*ChA) en démarrant à partir de mélanges d'isomères dudit acide (ChA), comprenant l'utilisation d'énantiomères du 2-diméthylamino-1-phényl-1,3-propanediol (DMPP) et du 2-diméthylamino-1-[4-(méthylthio)phényl]propane-1,3-diol (MTDP) en tants que sélecteurs chiraux.

2. Procédé selon la revendication 1, pour la séparation du *cis*-ChA à partir du *trans*-ChA, comprenant la réaction de ChA avec du *1S,2S*-(+)-DMPP ou avec du *1R,2R*-(-)-DMPP, utilisé dans une quantité de 1 équivalent par rapport à la quantité de *trans*-ChA présente dans ledit ChA.

3. Procédé selon la revendication 1, pour la séparation d'un mélange racémique de *trans*-ChA en ses énantiomères d-*trans* et l-*trans*, comprenant la réaction dudit mélange avec du *1S,2S*-(+)-MTDP ou du *1R,2R*-(-)-MTDP dans une quantité de 0,5 équivalent par rapport au *trans*-ChA présent dans le mélange.

4. Procédé selon la revendication 1, pour la séparation d'un mélange scalémique de *trans*-ChA, comprenant la réaction dudit mélange avec du *1S,2S*-(+)-DMPP ou avec du *1R,2R*-(-)-DMPP.

5. Procédé selon la revendication 4, dans lequel ledit mélange scalémique est enrichi en énantiomère d, et où du *1S,2S*-(+)-DMPP est utilisé dans une quantité de 2 équivalents par rapport à l'énantiomère 1 moindre, ou du *1R,2R*-(-)-DMPP est utilisé dans une quantité de 1 équivalent par rapport à l'excès d'énantiomère d.

6. Procédé selon la revendication 4, dans lequel ledit mélange scalémique est enrichi en énantiomère 1, et où du *1S,2S*-(+)-DMPP est utilisé dans une quantité de 1 équivalent par rapport à l'excès d'énantiomère 1, ou du *1R,2R*-(-)-DMPP est utilisé dans une quantité de 2 équivalents par rapport à l'énantiomère d moindre.

7. Procédé selon les revendications 1 à 6, dans lequel le produit réactionnel avec du DMPP et du MTDP est un sel de type *p*₁*n*₁ ou de type *n.*

8. Procédé selon la revendication 7, dans lequel l'énantiomère requis de *trans*-ChA est récupéré à partir du sel *p*₁*n*₁ par dissolution du sel dans une solution d'acide, extraction avec un solvant organique, récupération et évaporation de la phase organique, dissolution du solide résultant de l'évaporation dans un solvant approprié et réaction de la solution résultante avec du *1S,2S*-(+)-MTDP ou du *1R,2R*-(-)-MTDP dans une quantité de 0,5 équivalent par rapport au *trans*-ChA présent.

9. Procédé selon la revendication 7, dans lequel l'énantiomère requis de *trans*-ChA est récupéré à partir dudit sel n par dissolution du sel dans une solution d'acide, extraction avec un solvant organique, récupération et évaporation de la phase organique.

10. Procédé selon la revendication 7 ou 8, dans lequel la solution aqueuse demeurant après l'acidification et l'extraction est basifiée, la récupération du DMPP ou du MTDP présent dans la solution sous la forme d'un précipité sous une forme pure sur le plan énantiomère, avec possibilité de réutilisation dans des cycles de salification ultérieurs.

11. Procédé selon les revendications 1 à 10, dans lequel les liqueurs mères obtenues à partir des réactions avec du DMPP ou du MTDP sont en outre traitées pour récupérer les énantiomères requis à partir du ChA restant en solution.

12. Procédé selon la revendication 10, dans lequel lesdites liqueurs mères contiennent le diastéréo-isomère *cis-*ChA et où l'énantiomère requis (d-*trans* ou l-*trans*) est obtenu par conversion du *cis*-ChA en *trans*-ChA par traitement avec des acides de Lewis et traitement du *trans*-ChA résultant au moyen de la procédure décrite dans les revendications 3 à 9.

13. Procédé selon la revendication 10, dans lequel lesdites liqueurs mères contiennent le diastéréo-isomère *trans*-ChA et où l'isomère requis (d-*trans* ou l-*trans*) est récupéré en suivant la procédure décrite dans les revendications 3 à 9.

14. Procédé selon la revendication 10, dans lequel lesdites liqueurs mères contiennent l'énantiomère d-*trans*-ChA ou l'énantiomère l-*trans*, ledit énantiomère d ou 1 étant récupéré par évaporation des liqueurs mères respectives jusqu'à l'état sec.

15. Procédé selon les revendications 1 à 14, dans lequel le mélange des isomères du ChA à séparer est **caractérisé par** un rapport *trans*-ChA/*cis*-ChA entre 30/70 et 99/1.

16. Procédé selon les revendications 1 à 14, dans lequel le mélange des isomères du ChA à séparer est **caractérisé par** un rapport *trans*-ChA/*cis*-ChA de 65/35, 80/20 ou 92/8.

17. Procédé selon la revendication 2, dans lequel toutes les réactions de salification avec du DMPP ou du MTDP sont entreprises dans de l'éther isopropylique.

18. Sel de l'acide chrysanthémique avec du *1S,2S*-(+)- ou du *1R,2R*-(-)-DMPP, choisi parmi :
le dl-*trans*-ChA•*1S,2S*-(+)-2-diméthylamino-1-phényl-1,3-propanediol ;
le dl-*trans*-ChA•*1R,2R*-(-)-2-diméthylamino-1-phényl-1,3-propanediol ;
le l-trans-ChA•*1S,2S*-(+)-2-diméthylamino-1-phényl-1,3-propanediol ;
le l-trans-ChA•*1R,2R*-(-)-2-diméthylamino-1-phényl-1,3-propanediol ;
le d-trans-ChA•*1S,2S*-(+)-2-diméthylamino-1-phényl-1,3-propanediol ;
le d-trans-ChA•*1R,2R*-(-)-2-diméthylamino-1-phényl-1,3-propanediol.

19. Sel de l'acide chrysanthémique avec du *1S,2S*-(+)- ou du *1R,2R*-(-)-MTDP, choisi parmi :
le l-*trans*-ChA•*1S,2S*-(+)-2-diméthylamino-1-[4-(méthylthio)phényl]propane-1,3-diol ;
le d-*trans*-ChA•*1R,2R*-(-)-MTDP-2-diméthylamino-1-[4-(méthylthio)phényl]propane-1,3-diol.

20. 2-Diméthylamino-1-[4-(méthylthio)phényl]propane-1,3-diol.
